# EUROPÄISCHE PATENTSCHRIFT

(11) **EP 3 846 933 B1**
(45) Veröffentlichungstag und Bekanntmachung des Hinweises auf die Patenterteilung: **07.05.2025**
(21) Anmeldenummer: 19766011.1
(22) Anmeldetag: 09.09.2019
(51) Int. Cl.: B01J 20/00, C02F 1/28, C02F 1/00, B01D 29/58, C02F 101/20, B01J 49/85, B01J 47/024, B01J 47/022, B01J 45/00, B01J 20/28, B01J 20/20

(54) **VORRICHTUNG ZUR MEHRSTUFIGEN REINIGUNG VON TRINKWASSER**
DEVICE FOR PURIFYING DRINKING WATER IN MULTIPLE STAGES
DISPOSITIF POUR UNE PURIFICATION EN PLUSIEURS ÉTAPES D'EAU POTABLE

(30) Priorität: 07.09.2018 DE 102018121904
(43) Veröffentlichungstag der Anmeldung: 14.07.2021
(73) Patentinhaber: instrAction GmbH, 69124 Heidelberg (DE)
(72) Erfinder: WELTER, Martin, 69151 Neckargemünd (DE); MEYER, Christian, 68723 Schwetzingen (DE); LUNGFIEL, Kristian, 65195 Wiesbaden (DE)
(74) Vertreter: Wetzel, Fritz
(86) Internationale Anmeldenummer: PCT/EP2019/073952
(87) Internationale Veröffentlichungsnummer: WO 2020/049184

(56) Entgegenhaltungen:
- DE-A1- 102016 107 485
- DE-T2- 3 787 659
- DE-U1- 202016 100 447
- DE-U1- 202018 100 396

## Beschreibung

Die vorliegende Erfindung betrifft eine Vorrichtung, in der mindestens zwei Wasserreinigungsverfahren in einer Einheit kombiniert werden, wobei ein Verfahren ein chelatisierendes Gel und/oder ein bakterizides Gel zur Schwermetallentfernung und/oder Bakterienentfernung umfasst.

Auf dem Markt für Trinkwasserreinigung ist eine Vielzahl von Geräten verfügbar, die unterschiedliche Strategien und Techniken verfolgen und teilweise bereits kombinieren.

Neben der dominierenden Technik der Umkehrosmose mit dem größten Marktanteil, gibt es eine Vielzahl von Geräten, die unterschiedliche Filtrationstechniken bzw. Destillationsverfahren einsetzen.

Alle bekannten Verfahren haben (teilweise gravierende) Nachteile:
Bei der Umkehrosmose liegt der größte Nachteil in der geringen Ausbeute an Trinkwasser, das selten 10% des eingesetzten Wasservolumens übersteigt, energieintensiv ist und auch gesundheitsfördernde Elemente, wie Magnesium, aus dem Trinkwasser entfernt. Teilweise wird dieses aufwendig in einem zweiten Schritt dem Trinkwasser wieder zugeführt.

Den Destillationsverfahren ist der Nachteil des extrem hohen Energieverbrauchs gemein. Zudem werden auch dort, wie bei der Umkehrosmose, die gesundheitsfördernden Elemente entfernt, so dass destilliertes Wasser entsteht, dass für den dauerhaften Konsum nicht geeignet ist und wieder um wichtige Inhaltsstoffe wie Magnesiumsalze in einem darauffolgenden Schritt angereichert werden muss.

Die Wasserreinigungsmaschinen, die mehrere Filtertechniken in separaten Einheiten/Kartuschen kombinieren, benötigen eine aufwendige Verrohrung mit entsprechenden Ventilen oder Konnektoren/Verbindern, die der Natur nach störanfällig sind und die Möglichkeit für Leckagen etc. bieten. Zudem sind Verbindungen Orte, an denen Bakterien etc. aufgrund der Strömungsverhältnisse besonders gute Möglichkeiten des Wachstums haben.

Gegenüber der oben erwähnten RO-Technik und den Destillationsverfahren arbeiten viele Reinigungsmethoden, die auf Filtration beruhen (bzw. auf der Kombination von unterschiedlichen, orthogonalen Filtrationstechniken) mit in der Regel hoher (100%iger) Ausbeute und mit Leitungsdruck, so dass kein zusätzlicher Energieverbrauch entsteht. Voraussetzung dafür ist allerdings ein Aufbau mit geringem apparativem Druckabfall und die Verwendung grobkörniger Absorberharze, die die Effektivität der Abreicherung und die Produktivität mindern.

Ein bekanntes und auf dem Markt genutztes Filtermedium ist z. B. die Aktivkohle, die als Schüttung von Partikeln in linear durchspülten Kartuschen oder als verpresster Hohlzylinder mit radialer Durchspülung eingesetzt wird. Aus Sicht der Produktivität und des Druckabfalls stellt der Hohlzylinder den idealen Aufbau dar.

Andere bekannte Medien sind das MetCap^{®}-Harz (WO2016030021) zur Entfernung von Schwermetallen aus Trinkwasser und das BacCap^{®}-Harz (DE 102017007273A1) zur Entfernung von Bakterien aus Trinkwasser. Beide werden üblicherweise in Kartuschen verwendet und teilweise kombiniert.

Bei den MetCap-Harzen handelt es sich um ein lineares Polyvinylamin, das auf einen porösen Partikel aufgebracht und anschließend mit einem bi-funktionalen Vernetzer zu einem dreidimensionalen polymeren Netzwerk umgesetzt wird. Dieses Netzwerk verfügt über zahlreiche Aminogruppen in hoher Dichte und kann durch die Ausbildung sehr stabiler Metall-Amin-Komplexe chelatisierend Schwermetalle aus Lösungen mit hoher Kapazität binden und somit entfernen. Für Schwermetalle, die nur schwache Amin-Komplexe bilden (z.B. Nickel, Mangan), können weitere chelatisierende Gruppen in das polymere Netzwerk eingeführt werden, z.B. Carboxylate, Thiole etc.

Bei den BacCap-Harzen handelt es ebenfalls um Amino-Polymere, die in ähnlicher Weise hergestellt werden, wie die MetCap Absorber. Allerdings ist die Mischung, Stöchiometrie, Vernetzungsgrad etc. auf eine anti-bakterielle Wirkung hin optimiert. Die anti-bakterielle Wirkung beruht aller Wahrscheinlichkeit nach auf der Wechselwirkung zwischen den zumindest teilweise protonierten (und damit positiv gelandenen) Aminogruppen des Polymers mit der negativ polarisierten Bakterienhülle. Eine mögliche Erklärung ist eine direkte Interaktion der polymeren Aminogruppen mit den Fettsäuren der Hülle, die dadurch geschädigt wird. Eine zweite Erklärung könnte die Blockade der Ionenkanäle in den Zellwänden durch das Aminopolymer sein. Im Endeffekt führen beide Erklärungen zur Zerstörung oder Schädigung der Zellmembran und letztlich zu einem Absterben der Bakterien.

Dabei ist es besonders wichtig darauf hinzuweisen, dass weder zur Entfernung der Bakterien noch zur Bindung der Schwermetalle Substanzen an das Trinkwasser abgegeben werden. Darin unterscheidet sich das vorgeschlagene Verfahren deutlich von anderen, die Silber, Chlor oder andere Substanzen an das Trinkwasser abgeben und diese kontaminieren.

Ein weiterer Vorteil der vorgeschlagenen Vorrichtung erschließt sich durch die einfache Handhabung. Im Gegensatz zu
Filtrationsprozessen, wie der Umkehrosmose, ist keine Druckerhöhung durch eine Pumpe erforderlich, die wiederum Elektrizität benötigt. Gleiches gilt für UV-Systeme auf dem Markt, die ebenfalls während des Betriebs Strom benötigen.

Ein drittes Verfahren zur Trinkwasserreinigung ist die Filtration durch Micro- und Nanofiltrationsverfahren.

Ein weiteres Verfahren zur Behandlung, speziell zur Enthärtung von Trinkwasser, ist die Filtration des Kalzium- und Magnesiumhaltigen Trinkwassers über Ionenaustauscher. Dabei werden Kalzium und Magnesium gebunden und dafür jeweils zwei Mol-Äquivalente Natrium in das Trinkwasser abgegeben. Das Verfahren ist aufgrund der negativen Auswirkungen von zu viel Natrium auf Herz und Kreislauf in die Kritik geraten. Ein Nachteil dieses Verfahrens ist zudem die geringe Kapazität und die Notwendigkeit häufigen Austauschs oder Regenerierung. Zudem neigen diese Geräte zur Verkeimung.

Im großen Gegensatz zu den drei erstgenannten Filtermedien ist die Filtrationskapazität des Ionenaustauschers bei weitem am schnellsten erschöpft. Beim Einsatz harten Wassers ist ein Austausch bzw. eine Regeneration des Harzes bereits nach wenigen Tagen, spätestens aber nach ein bis zwei Wochen bei ordnungsgemäßem Einsatz erforderlich.

Kartuschen, gefüllt mit Schwermetall-absorbierenden Harzen (z.B. MetCap^{®}), Bakterien-entfernden Harzen (z.B. BacCap^{®}), Aktivkohle oder auch die Filtrationsmembranen haben eine Lebensdauer von ca. 6 Monaten. Nicht selten müssen diese Elemente vor ihrer technischen Erschöpfung sicherheitshalber ausgetauscht werden. Neben den oben beschriebenen Vorrichtungen gibt es eine ganze Reihe von Geräten auf dem Markt bzw. sind geschützt, die die einzelne Reinigungstechniken modulartig miteinander kombinieren.

Diese Geräte haben den Vorteil, dass die Kunden individuell je nach Charakter und Belastung ihres Trinkwassers, das regional sehr verschiedene Verunreinigungen in unterschiedlicher Konzentration aufweisen kann, die jeweiligen Reinigungskartuschen bei Erschöpfung einzeln austauschen können. Nachteilig ist, dass zur Überwachung Kapazität jeder einzelnen Kartusche Sensoren eingebaut werden müssen, die individuell Meldungen ausgeben, wenn Teile der Anlage erschöpft sind. Alternativ geben Hersteller Protokolle heraus, die bestimmte Zeiträume zum Wechsel der einzelnen Kartuschen vorgeben.

Das Verfahren ist aufwendig und verbraucher-unfreundlich. Es besteht die Gefahr, dass die aufwendigen Protokolle oder Wartungszeiträume nicht eingehalten werden und die Trinkwasserqualität im Durchschnitt eher leidet, als eine Verbesserung erfährt.

DE202018101926U1 offenbart eine zweiteilige Filtervorrichtung für den Reinigung von Wasser in Luftfahrzeugen. Die Druckschrift beschreibt einen äußeren Hohlzylinder aus Aktivkohle mit innen liegenden Membranen (speziell Hohlfiltermembranen). Das zu filtrierende Wasser wird seitlich in die Aktivkohle eingeführt, durch diese filtriert und gelangt schließlich in den Innenraum des Aktivkohlehohlzylinders. Die Porengröße der Aktivkohle beträgt bevorzugt 0,5 µm. Die Porengröße der innenliegenden Membran liegt bevorzugt in einem Bereich um 0,2 µm. Diese verhältnismäßig großen Poren wurden gewählt, um den Rückdruck des Systems gering zu halten. Die Autoren behaupten mit der von ihnen beanspruchten Vorrichtung Bakterien und Schwermetalle zurückhalten zu können.

Bekanntermaßen werden Membranen, speziell Hohlfasermembranen zur Wasserfiltration in einem weiten Bereich eingesetzt. Zur Bakterienentfernung kommen in der Regel Membranen mit einer Porenweite von 0,02 µm (20 nm) zum Einsatz, da größere Porenweiten Bakterien und andere Keime (insbesondere Viren)
nicht zurückhalten. Ein deutlicher Druckanstieg wird dabei in Kauf genommen, um den Bakterienrückhalt sicher zu stellen. Die in der vorliegenden Druckschrift vorgeschlagenen 200 nm liegen deutlich über den üblichen 20 nm. Ein signifikanter Bakterienrückhalt scheint damit sehr unwahrscheinlich. Vermutlich wurde die große Porenweite aus Gründen der Rückdruckminimierung gewählt.

Das aufgeführte Beispiel der Abreicherung wird anhand von Brevundimonas diminuta demonstriert. Dieser Keim ist in keiner Trinkwasserverordnung gelistet und eher ein exotischer Keim. Üblicherweise werden E. coli oder Pseudomonas aeruginosa als Testorganismus verwendet.

Die Aktivkohle selbst ist als Bakterienrückhalt weitgehend ungeeignet. Zwar werden bei hinreichend kleinen Poren Bakterien zunächst zurückgehalten. Was allerdings aufgrund der 0,5 µm großen Poren der Aktivkohle bereits fraglich ist. Anschließend wachsen die Bakterien in der Aktivkohle und werden dann ins Wasser abgegeben, wobei dieses vekeimt wird. Vor diesem Hintergrund ist eine zweite Reinigungsstufe durchaus sinnvoll. Werden dort die Poren allerdings so groß gewählt, wie oben angegeben, scheint eine effektive Filtration sehr fraglich. Gleiches gilt für die Entfernung von Schwermetallen. Schwermetalle werden durch Aktivkohle gar nicht oder nur zu einem sehr geringen Anteil mit sehr geringer Kapazität entfernt. Durch Ultrafiltrationsmembranen werden Schwermetalle keinesfalls aufgehalten. Dazu sind Membranen mit Porenweiten im einstelligen Nanometer-Bereich erforderlich, die sich durch ihren hohen Druckabfall für die intendierte Anwendung nicht eignen.

Im Gegensatz zu der in DE202018101926U1 aufgeführten Vorrichtung mit reinen Kombinationen von Aktivkohle und Membranfiltration wird in der vorliegenden Anmeldeschrift die Kombination mit speziell für die Trinkwasserreinigung entwickelten Absorbergelen vorgeschlagen. Speziell das von instrAction entwickelte chelatisierende Absorbegel bindet Schwermetalle effektiv, schnell und mit hoher Kapazität. Diese Performance lässt sich durch einfache Membranfiltration nicht erreichen. Gleichzeitig entfernen die Partikel mit anti-bakterieller Wirkung im vorgeschlagenen Aufbau effektiv Trinkwasser-relevante Keime, insbesondere Bakterien durch Filtration - im Gegensatz zu Aktivkohle, die eher als Bakterienquelle gilt und Filtermembranen mit zu großer Porenweite.

In DE10217649A1 wird ein Verfahren vorgestellt, in dem eine Edelmetalloberfläche so behandelt wird, dass sie, sobald sie mit Wasser in Kontakt gebracht wird, Metall-Ionen an das Wasser abgeben, die ihrerseits dann Bakterien abtöten. Als bevorzugtes Edelmetall wird unter anderem Silber vorgeschlagen. Das Wirkprinzip beruht also auf der Abgabe von anti-bakteriellen Stoffen. Es ähnelt damit der in der Kritik stehenden Silberung von Ionenaustauschern zum gleichen Zweck. Diesem Verfahren ist die Freigabe von potentiell gesundheitsschädlichen Metallen eigen. Fernerhin wird die Entstehung von Silber-resistenten Keimen als Nachteil angesehen. Schwermetalle werden mit diesem Verfahren nicht entfernt, im Gegenteil: Am Ende finden sich im Filtrat (erfindungsgemäß) mehr Schwermetalle als zuvor.

Die vorliegende Erfindung beruht auf der Wechselwirkung der Bakterien mit der Partikeloberfläche in der vorgeschlagenen Vorrichtung und nicht auf der Freisetzung von anti-bakteriell wirkenden Schwermetall-Ionen in das Trinkwasser. Durch das vorgeschlagene Verfahren werden Bakterien und andere Keime nicht nur wirkungsvoll aus dem Trinkwasser gefiltert, sondern zudem auch noch Schwermetalle entfernt.

In CH339888A wird eine Filterkerze aus einer Aktivkohleschüttung mit einem inneren zentralen Ablauf zur Wasserreinigung vorgeschlagen. Die Aktivkohle wird dabei mit "oligodynamisch wirksamen Substanzen", wie Silber oder Kupfer bzw. die Salze dieser Metalle "verbunden oder imprägniert". Diese Substanzen töten verlässlich Bakterien ab. Dadurch soll ein Durchwachsen von Keimen durch die Aktivkohle und eine Kontamination des filtrierten Wassers mit Bakterien verhindert werden. Diese Gefahr ist der wesentliche Kritikpunkt bei der Verwendung von Aktivkohle in der Wasserreinigung. Die gesamte Vorrichtung dient im Wesentlichen dazu, "Chlor" und andere negative Geschmacksstoffe über Absorption zu entfernen. Die Vorrichtung als solche wurde mittlerweile weiterentwickelt und kommt heute in der Regel als verpresste Aktivkohle in den Handel. Die in CH339888A vorgeschlagene Erfindung hat den gravierenden Nachteil, dass toxische Schwermetalle wie Silber und Kupfer an das Filtrat abgegeben werden.

Wie oben dargestellt entfällt dieser gravierende Nachteil beim Einsatz der vorliegenden Erfindung: Bei der beschriebenen erfindungsgemäßen Vorrichtung werden Bakterien durch Wechselwirkung mit der Partikeloberfläche des anti-bakteriellen Harzes entfernt. Substanzen, insbesondere Schwermetalle werden dabei nicht ins Filtrat abgegeben. Im Gegenteil werden Schwermetalle durch die Filtration des Wassers über das chelatisierende Harz entfernt.

In DE3001674A1 wird ein Filter vorgeschlagen, der Aktivkohle und einen Ionenaustauscher enthält. Der pH-Wert des zu reinigenden Wassers wird im Filter auf ca. pH = 3 abgesenkt, wodurch das Keimwachstum im Filter reduziert werden soll bzw. schon vorhandene Keime abgetötet werden. Gleichzeitig werden biozid wirkende Substanzen in die Lösung abgegeben, um Keime abzutöten bzw. weiteres Wachstum zu verhindern.

Wie in den Druckschriften zuvor, wird auch in dieser versucht, dem großen Problem der verkeimenden Aktivkohle durch die Freisetzung biozider Substanzen entgegenzuwirken. Die Veruneinigung des Filtrats mit diesen potentiell gesundheitsschädlichen Substanzen wird in Kauf genommen.

Diese Nachteile treten wie oben beschrieben bei Verwendung der erfindungsgemäßen Vorrichtung nicht auf.

In DE202018100396U1 wird ein modulares Wasserreinigungssystem beschrieben, in dem zwei bis fünf unterschiedliche Reinigungsverfahren modular miteinander kombiniert werden. Die individuellen Reinigungsstufen adressieren jeweils eine andere Gruppe von Kontaminanten, wie sie in Trinkwasser vorkommen können. Die einzelnen Module sind unabhängig voneinander und stehen durch eine Verrohrung miteinander in Kontakt. Die Module sind individuell austauschbar und können, sobald ihre Kapazität erschöpft ist, einzeln ersetzt werden. Der modulare Aufbau hat Vorteile insofern, dass einzelne Komponenten bei Vorliegen der entsprechenden Notwendigkeit getauscht werden können. Nachteilig ist die aufwendige und in vielerlei Hinsicht anfällige Verrohrung. Zudem ist die Entfernung von Bakterien durch einfache Filtration in DE202018100396U1 noch nicht vorgesehen.

Der aktuelle Vorschlag kombiniert zwei bis drei der langlebigsten Reinigungsverfahren in einer Kartusche in einer Weise, dass alle Reinigungsstufen in einem Modul konzentriert sind. Dies ist ein sehr Platz-sparendes und kundenfreundliches Verfahren, der nur noch eine Kartusche überwachen und austauschen muss, im Gegensatz zu den zwei bis fünf, wie in DE202018100396U1 vorgeschlagen. Fernerhin werden die Verfahren in einer dreidimensionalen Anordnung in eine Weise kombiniert, dass sie nur einen geringen Rückdruck verursachen; in DE202018100396U1 ist das nicht angelegt.

DE 20 2016 100447 U1 offenbart einen Filter, der ein elektroaktives Filtermaterial in einer ersten Lage und einer zweiten Lage umfasst. Das Gebrauchsmuster offenbart ferner eine dritte Lage, welche sich zwischen der ersten und zweiten Lage befindet.

Die erste und zweite Lage weist in wässriger Umgebung ein ZETA-Potenzial auf, wobei sich Viren und Keime durch Elektroadsorption anlagern können. Die dritte Lage enthält ein Adsorbens (Aktivkohle oder Zeolith), an dessen Oberfläche sich im Wasser befindliche Partikel oder gelöste Stoffe durch physikalische Adsorption (Van der Waals Bindung) anlagern.

DE 37 87 659 T2 offenbart einen Filter zum Entfernen von Verunreinigungsstoffen aus Wasser, wobei die Filteranordnung einen Faserfilter zum Beseitigen von festen Verunreinigungen aus dem Wasser, ein Sorptionsmittelbett zum Entfernen von chemischen Verunreinigungsstoffen aus dem Wasser stromabwärts von dem Faserfilter und ein Teil zum Entfernen von mikrobiologischen Verunreinigungsstoffen aus dem Wasser aufweist und stromabwärts von dem Faserfilter und dem Sorptionsmittelbett angeordnet ist. Der Faserfilter und das Sorptionsmittelbett sind zylindrisch angeordnet und ermöglichen eine radiale Wasserströmung. Der Bestandteil zur Eliminierung der mikrobiologischen Verunreinigungen ist durch eine mikroporöse Membran gekennzeichnet, die in der Lage ist, solche Verunreinigungen zu Filtern.

Keine der aufgeführten Druckschriften kombiniert einen starren Aktivkohle-Hohlzylinder mit einer partikulären Schüttung aus chelatisierenden und anti-bakteriellen Harzen. Eine derartige Kombination war bisher im Stand der Technik nicht bekannt. Gleiches gilt für den Aufbau mit zentralem Ablauf über das gesamte Modul hinweg, der in CH339888A ebenfalls vorgesehen ist. Dort werden allerdings nur inakzeptable Vorkehrungen zur Keimreduktion vorgeschlagen. Der innovative Ansatz, mehrstufiger, radialer Filter mit anti-bakteriellen Harzen ist in keiner der Druckschriften vorgesehen oder auch nur angelegt.

Vor diesem Hintergrund ergibt sich die Aufgabe, die Vorteile der bekannten Filtrationsverfahren in einer Weise zu kombinieren, dass die Nachteile des modularen Aufbaus minimiert werden bzw. gar nicht erst auftreten.

Die Aufgabe wurde gelöst durch eine Vorrichtung wie in Anspruch 1 definiert. Vorteilhafte Ausgestaltungen sind Gegenstand der abhängigen Ansprüche.

Gegenstand der vorliegenden Erfindung ist eine Vorrichtung zur mehrstufige Reinigung von Trinkwasser durch Kombination orthogonaler Reinigungstechniken in einem Modul, dadurch gekennzeichnet, dass die Vorrichtung ein Gehäuse (3), eine Wassereintrittsöffnung (1), eine Wasseraustrittsöffnung (2), einen mit Aktivkohle gefüllten äußeren Hohlzylinder (4) sowie einen inneren Hohlzylinder mit semipermeabler Wandung (5) umfasst, wobei der innere Hohlzylinder (5) ein chelatisierendes, und/oder ein bakterizides Gel zur Schwermetallentfernung und/oder Bakterienentfernung umfasst, wobei das chelatisierende Gel bzw. das bakterizide Gel oder beide zwischen einem äußeren Aktivkohlehohlzylinder und einem zentralen Ablauf über die gesamte Länge des Hohlzylinders (5) gefüllt sind, wobei der zentrale Ablauf aus einem mit einer Membran umwickelten Rohr mit größeren Öffnungen als dem Partikeldurchmesser des sie umgebenen Gels mit chelatisierender und/oder bakterizider Wirkung besteht und wobei die Membran über kleinere Poren verfügt, als der Partikeldurchmesser des sie umgebenden Gels mit chelatisierender und/oder bakterizider Wirkung.

Vorteilhafterweise kann das Gehäuse (3), die Wassereintrittsöffnung (1), die Wasseraustrittsöffnung (2), der äußere Hohlzylinder (4) sowie der innere Hohlzylinder (5) der Vorrichtung im 3D-Druck hergestellt werden. Dies ermöglicht eine kostengünstige Produktion und zudem können Form und Dimension der Vorrichtung für den Anwender maßgeschneidert werden.

In der beanspruchten Vorrichtung werden die mindestens zwei der langlebigen Filtrationsmedien bzw. -techniken miteinander kombiniert: Aktivkohle, ein Schwermetall-bindendes Absorberharz und/oder ein Bakterien-entfernendes Harz, sowie ggf. die Ultrafiltration.

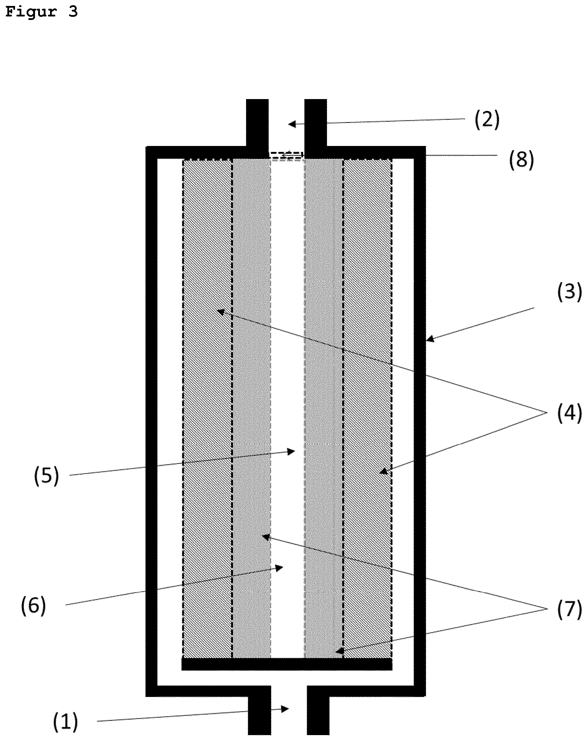

Dies geschieht, indem bekannte Aktivkohle-Hohlzylinder (4) in einem Gehäuse (3) mit Schwermetall-bindenden und/oder Bakterien-entfernenden Harzen(7) befüllt werden (Figur 1, Figur 3). Zentral wird ein Ablauf in Form eines Hohlzylinders mit semipermeabler Wandung (5) oder einer Hohlfasermembran oder eines Bündels von Hohlfasermembranen (6), bevorzugt über die gesamte Länge des Hohlzylinders eingeführt, so dass insgesamt mindestens zwei konzentrisch, nacheinander durchströmte Schichten mit zentriertem Ablauf dargeboten werden.

Insgesamt durchläuft das zu filtrierende Wasser damit zunächst den äußeren Aktivkohlehohlzylinder, gefolgt von dem inneren Hohlzylinder gefüllt mit Schwermetall-entfernenden Absorberharz und/oder Bakterien-entfernendem Harz. Zuletzt passiert das Wasser den zentralen Ablauf, der sich über die gesamte Länge der beiden Hohlzylinder erstreckt. Dieser kann - als dritte Reinigungsstufe - als Ultrafiltrations-Hohlfasermembran ausgeführt sein.

Der Wassereinlass (1) kann für einen einfachen Austausch/Anschluss an ein Wasserreinigungsgerät auf der gleichen Seite wie der Wasserauslass (2) angebracht sein (Figur 1, Figur 2), oder gegenüberliegend für einen linearen Einbau in ein Leitungssystem (Figur 3, Figur 4).

Der zentrale Ablauf (5) ist essentiell hinsichtlich einer optimalen Durchströmung der Absorbermaterialien bei gleichzeitigem geringem und gleichmäßigem Druckabfall über die gesamte Filterlänge.

Bei einer linearen Durchströmung der Filtermedien entsteht ein zu hoher Rückdruck, der entweder eine zusätzliche Pumpe erforderlich macht oder die Produktivität in inakzeptabler Weise senkt.

Wählt man zur Reduktion des Drucks zu große Partikel, wird die Produktivität aufgrund des langsamen Austauschs, der langen Diffusionsstrecke, zwischen kontaminiertem Wasser und den Bindeplätzen innerhalb des Absorbermaterials herabgesetzt. Wählt man zu geringe Betthöhen und reduziert damit die Verweildauer des Wassers im Absorberbett, resultiert daraus eine unzureichende Abreicherung der Kontaminanten.

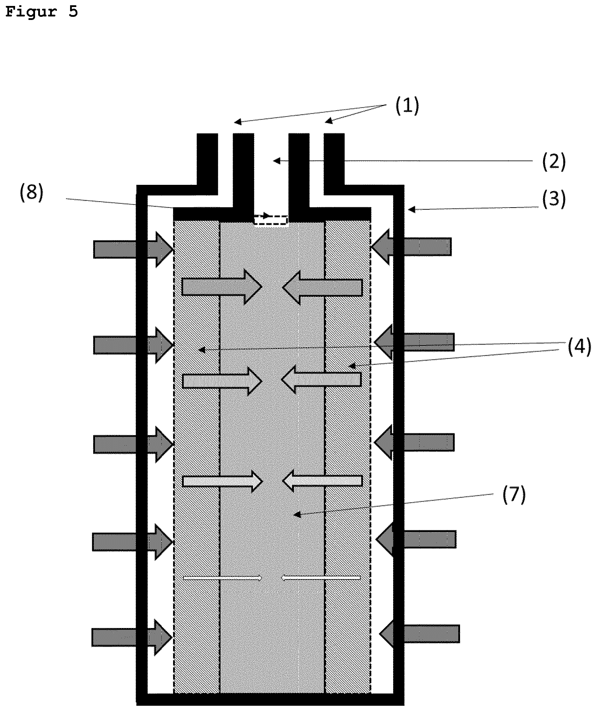

Füllt man den inneren freien Aktivkohlehohlzylinder (5) mit einem weiteren Absorbermaterial (6), ohne den hier beanspruchten zentralen Ablauf, erhält man einen Druckgradienten über die Länge des Hohlzylinders, der eine gleichmäßige Durchströmung des Gelbettes verhindert (7) und zu einer unzureichenden Abreicherung der Kontaminanten führt. Spätestens nach Erschöpfung der Kapazität am "kürzesten Weg" findet keine oder nur noch eine unzureichende Reinigung des Wassers statt (siehe Figur 5).

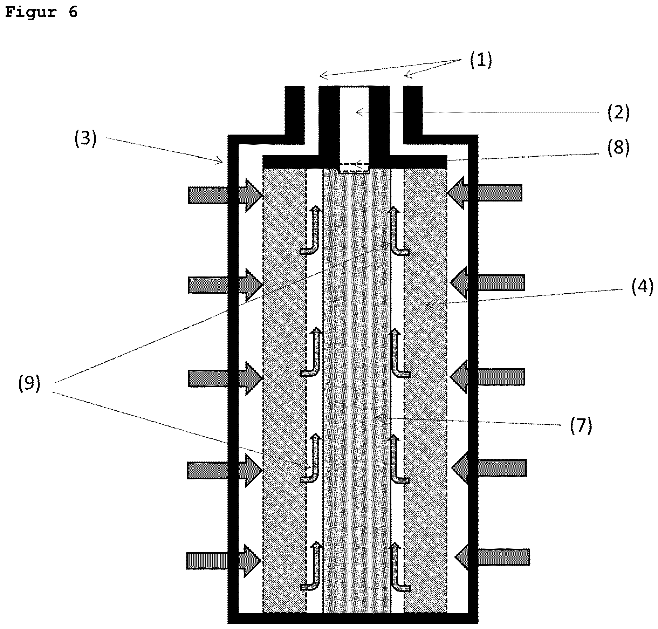

Bei einem gefüllten Hohlzylinder mit einfachem Ablauf an einer Seite des Zylinders können zudem Kanäle an der Wand des Einlaufs (Figur 6) entstehen (7), die eine Durchströmung der 30 Absorberpartikel ebenfalls verhindern und für keine oder eine zu geringe Abreicherung der Verunreinigungen aufgrund unzureichenden Kontakts zwischen Wasser und Absorber sorgen.

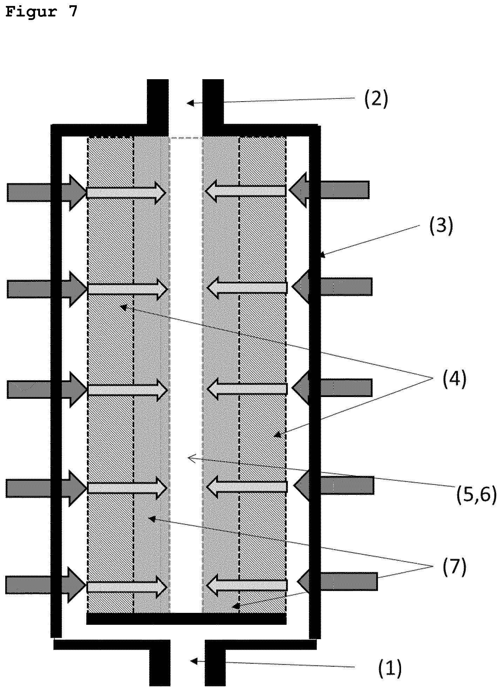

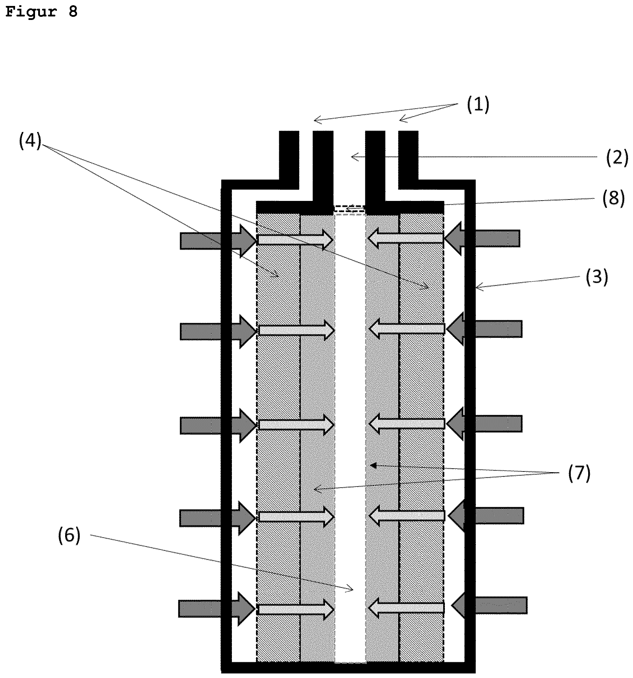

Ein Ausweg bietet hier die radiale Anordnung der Trennmedien (5) und (7) wie es in kommerziell verfügbaren Hohlzylinder mit Aktivkohleblöcken bereits realisiert ist, mit zentralem Ablauf (5) (Figur 7 und Figur 8). Dieser Aufbau erlaubt hohe Durchflussraten bei geringem Rückdruck, kurzer Trennstrecke und homogener, gleichmäßiger und vollständiger Durchströmung (8) bei gleichzeitig ausreichender Verweildauer des Wassers im Absorberbett.

Der Ablauf kann aus einem mehrfach durchbrochenen Rohr (6) mit entsprechend kleinen Öffnungen bestehen, die das filtrierte Wasser ohne nennenswerten Druckabfall durchlassen, das Harz aber zurückhalten.

Des Weiteren ist das zentrale Rohr mehrfach mit relativ zum Partikeldurchmesser des Harzes großen Öffnungen versehen und es ist zusätzlich mit einem geeigneten Filtertuch mit entsprechend kleiner Maschenweite versehen (6).

Weiterhin kann der zentrale Ablauf durch ein oder mehrere (gebündelte) Hohlfasermembranen erreicht werden, die sich über die gesamte Länge des Zylinders erstrecken (6).

Die Anordnung kann mit gegenüberliegendem Ein- und Auslass für den linearen Einbau in ein Leitungssystem (Figur 7) oder mit nur einem Anschluss für Ein- und Auslauf für einfachen Einbau in eine Wasserreinigungsmaschine ausgelegt sein (Figur 8).

Als eine nicht erfindungsgemäße Variante kann auch ein kombinierter Hohlzylinder verwendet werden, bei dem Aktivkohle und ein oder mehrere Absorberharze miteinander in geeigneter Weise verpresst/verklebt sind.

Die Mengen bzw. Volumina an Aktivkohle, Absorberharz bzw. die Menge und Kapazität der zentralen Ableitung bzw. der Membran kann auf die Ansprüche der Trinkwasserqualität abgestimmt und in einer Weise kombiniert werden, dass ein Maximum an Produktivität und Effektivität der Reinigung bei minimiertem Druckabfall erreicht wird.

Dieser Aufbau erlaubt eine Anpassung auf regionale Unterschiede und Trinkwassermärkte unter Beibehaltung des hier beanspruchten Prinzips.

Die beanspruchte Vorrichtung kombiniert mindestens zwei langlebige Wasserreinigungsverfahren in einer Kartusche, die einen extrem breiten Bereich möglicher Trinkwasserkontaminaten abdecken ("Chlor", kleine organische Moleküle, Arzneimittelrückstände, Schwermetalle, Bakterien, Viren, Partikel etc.).

Dabei sind die Reinigungselemente so angeordnet, dass eine optimale Durchströmung (und damit ein optimaler Wasser-Absorber-Kontakt) bei reduziertem Druckabfall erreicht wird.

Dieser Aufbau erlaubt eine hohe Produktivität (große Anströmfläche und kleine Partikeldurchmesser sind möglich) bei maximaler Reinigungseffizienz, die mit alternativen Aufbauten nicht erreicht werden kann.

Gleichzeitig wird eine kompakte Einheit mit minimalem 20 Platzbedarf erreicht, die vom Verbraucher leicht überwacht werden kann.

Die Kombination verschiedener (langlebiger) Reinigungstechniken verringert den Aufwand bei der Konstruktion und beim Gebrauch der entsprechenden Maschine (weniger bis keine Leitungen oder Adapter, nur noch ein bis zwei Anschlüsse etc.).

Auch ein einfaches Anschließen an einen Wasserhahn (ggf. über einen flexiblen Adapter) oder den Einbau in entsprechende Wasserleitungen ist denkbar.

Trotz des äußerlich linearen Aufbaus handelt es sich um eine radiale Filtration mit kurzen Filtrationswegen, ausreichender Verweildauer des Wassers im Gelbett und sehr einfachem Aufbau.

Die Handhabung für den Endnutzer ist verglichen mit einem modular aufgebauten System stark vereinfacht (Tausch/Überwachung nur noch einer Kartusche, statt zwei oder drei); gleiches gilt für Herstellung, Handel, Marketing, Vertrieb, Lagerhaltung etc.

Die Kartusche kann in einer bevorzugten Ausführungsform linear in eine Wasserleitung oder über einen einzigen Anschluss, wie er gebrauchsüblich auf dem Markt bereits verwendet wird, eingebaut werden.

Die Vorrichtung kann leicht mit allen gängigen weiteren Reinigungs- oder Lagerungsmodulen kombiniert werden, beispielsweise einem nachfolgenden Tank zur Aufbewahrung des gereinigten Wassers, oder weiteren Reinigungstechniken, wie UV-Entkeimung (im Tank oder On-Line), Redoxfiltern etc., bzw. zur weiteren Verwendung in der Heißwasserbereitung, CO2-Zusatz-Modul zur Herstellung von Sprudelwasser, etwaige Chlorierung oder Wasserstoffperoxid-Zusatz zur nachträglichen Entkeimung oder Haltbarmachung, Zusatz von gesundheitsfördernden Ionen, wie Calcium und/oder Magnesium etc.

Die Vorrichtung beeinflusst oder beeinträchtigt die Art der nachfolgenden Wasserentnahme oder Wasserbehandlung nicht.

Die Leistungskraft der Vorrichtung kann an geeigneter Stelle, entweder am Entnahmepunkt oder an den Punkten zwischen den einzelnen Modulen durch geeignete Sensoren überwacht werden. Geeignete Sensoren sind beispielsweise, aber nicht ausschließlich, pH-Sensoren, Leitfähigkeitssensoren, Sensoren zur Kontrolle der Bakterienkonzentration, Ionen-selektive Sensoren, UV-Sensoren etc. Eine Durchflusszelle kann die prozessierte Wassermenge messen.

Die Sensoren sind in einer bevorzugten Ausführungsform an ein Datenverarbeitungssystem angeschlossen, dass aufgrund der Messwerte die Funktion der einzelnen Module überwacht und entsprechende Meldungen ausgibt, wenn der Austausch oder die Regeneration einer Kartusche zu erfolgen hat. Der Austausch der Module kann mit Hilfe der Sensoren auch rein Zeit-gesteuert bzw. Volumen-gesteuert erfolgen. Je nach Ausführungsform kann das Datenverarbeitungssystem eine automatische Regeneration des Enthärtungsmoduls einleiten oder ein Ventil verschließen, um den Austausch von Modulen als Voraussetzung für einen Weiterbetrieb zu erzwingen.

Das Datenverarbeitungssytem kann so programmiert sein, dass es bei Erschöpfung oder Fehlern eine Nachricht, z.B. an ein Mobilfunkgerät, E-Mail, SMS, Instant Massage etc. ausgibt, die den Verbraucher auf die Notwendigkeit des Kartuschentauschs hinweist.

In der kleinsten Ausführung eignet sich die Vorrichtung für den Einsatz im Haushalt und orientiert sich an den typischen Verbräuchen. In größeren Ausführungen kann die Vorrichtung auch in Mehrfamilie-Häusern, Wohnanlagen, in Restaurants, Krankenhäusern, auf Schiffen oder anderen Einrichtungen mit einem Bedarf an qualitativ hochwertigem Trinkwasser eingesetzt werden.

Die Kartusche selbst, d.h. das äußere Gehäuse (3), die Wassereintrittsöffnung (1), die Wasseraustrittsöffnung (2), der äußere Hohlzylinder aus Aktivkohle (4) sowie der innere zentrale Hohlzylinder mit einem Hohlfasermembranbündel (6) oder permeabler Wandung (6) sind bevorzugt in Kunststoff ausgeführt. Die Herstellung erfolgt nach etablierten Spritzgussverfahren oder im 3D-Druck, bzw. Kombinationen daraus. Eine Nachbearbeitung einzelner Elemente, beispielsweise das Durchführen von Bohrungen etc. ist ebenfalls vorgesehen. Die Hohlfasern selbst sind gewöhnlich aus Polyethersulfon-Polymeren (PES) aufgebaut. Sie können aber auch aus anderen Materialien bestehen.

### Figurenverzeichnis:

Figur 1: Längsschnitt der Doppelhohlzylinderkartusche mit einem Anschluss für Wassereinlass (1), Wasserauslass (2), Gehäuse (3), Hohlzylinder aus Aktivkohle (4), Holzylinder mit permeabler Wandung (5) oder Hohlfasermembranbündel (6), Schwermetall-bindenden chelatisierenden Harzen und/oder Bakterien-entfernenden Harz (7).

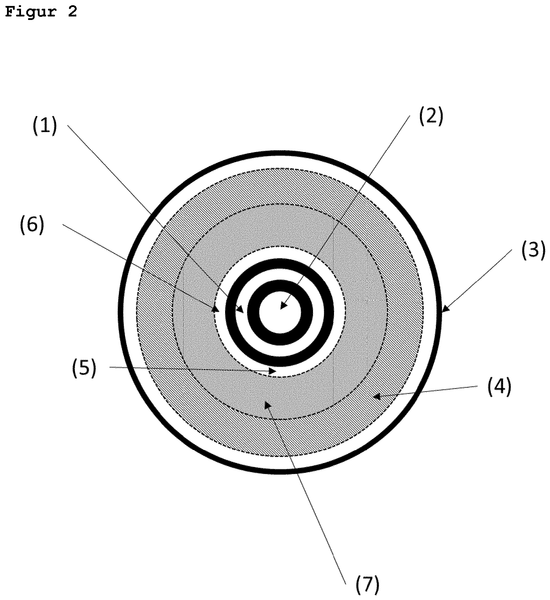

Figur 2: Querschnitt der Doppelhohlzylinderkartusche mit einem Anschluss für Wasserein- (1) und Austritt (2); Gehäuse (3), Aktivkohle (4), Schwermetall-bindendem chelatisierenden Harze und/oder Bakterien-entfernenden-Harz als Füllung (7), Hohlzylinder mit permeabler Wandung (5) oder einer oder mehrerer Hohlfasern (6).
Figur 3: Längsschnitt der Doppelhohlzylinderkartusche mit je einem Anschluss für Wasserein- (1) und Auslass (2) (linearer Aufbau); Gehäuse (3), Hohlzylinder aus Aktivkohle (4), Hohlzylinder mit permeabler Wandung (5) oder einer oder mehrerer Hohlfasern (6) Schwermetall-bindendem chelatisierenden Harz und/oder Bakterien-entfernenden Harz als Füllung (7), Fritte (8).

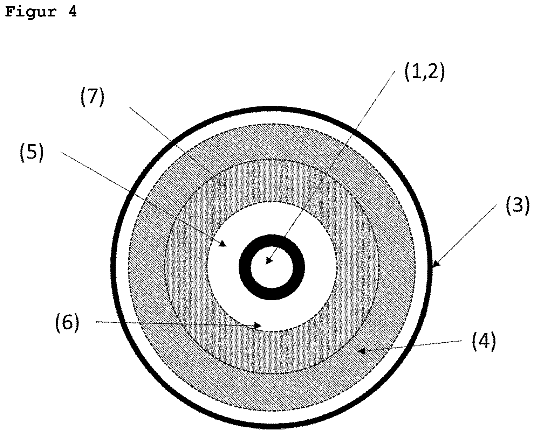

Figur 4: Querschnitt der Doppelhohlzylinderkartusche mit zwei Anschlüssen für Wasserein- (1) und Austritt (an der gegenüber liegenden Seite verdeckt), (linearer Aufbau), Gehäuse (3), Aktivkohlehohlzylinder (4), Schwermetall-bindende chelatisierende Harze und/oder Bakterien-entfernendes Harz als Füllung (7), Hohlzylinder mit permeabler Wandung (6) oder einer oder mehrerer Hohlfasern (5).
Figur 5: Unvorteilhafter Filtrationsweg des Wassers bei mit Absorbergel gefüllten Hohlfaserkartuschen ohne zentralen Ablauf aufgrund des geringeren Staudrucks am Kartuschenkopf (bevorzugte Fließrichtung durch Dicke der Pfeile in der Kartusche angedeutet), Wassereinlass (1), Wasserauslass (2), Gehäuse (3), Hohlzylinder aus Aktivkohle (4), Schwermetall-bindendem chelatisierenden Harze und/oder Bakterien¬entfernenden Harz in Bettform (7), Fritte (8).
Figur 6: Kanal-Bildung (Bypassing) des zu filtrierenden Wassers (9) bei einer Hohlfaserkartusche ohne zentralen Ablauf mit Wassereinlass (1), Wasserauslass (2), Gehäuse (3), Hohlzylinder aus Aktivkohle (4), Schwermetall-bindendem chelatisierenden Harze und/oder Bakterien¬entfernenden Harz als Füllung (7), Fritte (8) und Kanal-Bildung (Bypassing) (9).
Figur 7: Vorteilhafter Filtrationsweg bei gefülltem Hohlzylinder mit zentralem Ablauf und linearem Aufbau mit gegenüberliegendem Ein- (2) und Auslass (2); Wassereinlass (1), Wasserauslass (2), Gehäuse (3), Hohlzylinder aus Aktivkohle (4), Hohlzylinder mit permeabler Wandung oder Hohlfasermembranen (5,6), Schwermetall-bindendem chelatisierenden Harze und/oder Bakterien-entfernenden Harz als Füllung (7).
Figur 8: Vorteilhafter Filtrationsweg eines Hohlzylinders mit Ein- (1) und Auslauf (2) auf der gleichen Seite; Wassereinlass (1), Wasserauslass (2), Gehäuse (3), Hohlzylinder aus Aktivkohle (4), (6) Hohlzylinder mit permeabler Wandung oder Hohlfasermembranen (5,6), Schwermetall-bindendem chelatisierenden Harze und/oder Bakterien-entfernenden Harz als Füllung (7), Fritte (8).

## Patentansprüche

1. Vorrichtung zur mehrstufige Reinigung von Trinkwasser durch Kombination orthogonaler Reinigungstechniken in einem Modul, **dadurch gekennzeichnet**, das die Vorrichtung ein Gehäuse (3), eine Wassereintrittsöffnung (1), eine Wasseraustrittsöffnung (2), einen mit Aktivkohle gefüllten äußeren Hohlzylinder (4) sowie einen inneren Hohlzylinder mit semipermeabler Wandung (5) umfasst, wobei der innere Hohlzylinder (5) ein chelatisierendes, und/oder ein bakterizides Gel zur Schwermetallentfernung und/oder Bakterienentfernung umfasst, wobei das chelatisierende Gel bzw. das bakterizide Gel oder beide zwischen einen äußeren Aktivkohlehohlzylinder und einem zentralen Ablauf über die gesamte Länge des Hohlzylinders (5) gefüllt sind, wobei der zentrale Ablauf aus einem mit einer Membran umwickelten Rohr mit größeren Öffnungen als dem Partikeldurchmesser des sie umgebenen Gels mit chelatisierender und/oder bakterizider Wirkung besteht und wobei die Membran über kleinere Poren verfügt, als der Partikeldurchmesser des sie umgebenden Gels mit chelatisierender und/oder bakterizider Wirkung .

2. Vorrichtung nach Anspruch 1, **dadurch gekennzeichnet, dass** der zentrale Ablauf aus einem mehrfach durchbrochenen Rohr mit Öffnungen besteht.

3. Vorrichtung nach Anspruch 2, **dadurch gekennzeichnet, dass** die Öffnungen des mehrfach durchbrochenen Rohrs kleiner sind als die Partikel des sie umgebenen Gels mit chelatisierender und/oder bakterizider Wirkung.

4. Vorrichtung nach Anspruch 1, **dadurch gekennzeichnet, dass** der zentrale Ablauf aus einer oder mehreren Hohlfasermembranen oder einem Bündel von Hohlfasermembranen besteht.

5. Vorrichtung nach einem der Ansprüche 1 bis 4, **dadurch gekennzeichnet, dass** die Vorrichtung einen pH-Sensor, Leitfähigkeitssensor, UV-Sensor, oder Sensoren zur Bakterien-Bestimmung umfasst.

6. Vorrichtung nach Anspruch 5 **dadurch gekennzeichnet, dass** die Sensoren eine Warnung ausgeben, wenn definierte Grenzwerte über- oder unterschritten werden.

7. Vorrichtung nach einem der Ansprüche 1 bis 6, wobei die Vorrichtung weitere Elemente enthält, wobei die weiteren Elemente ausgewählt sind aus einem Wassertank, einer Enthärtungsanlage, einer Warmwasser-Bereitungsanlage, einer Anlage zur (UV)-Entkeimung, Redoxfilter, einer CO₂-Zugabeeinheit oder einer Chlorungseinheit.

8. Vorrichtung nach einem der Ansprüche 1 bis 7, wobei das Gehäuse (3), die Wassereintrittsöffnung (1), die Wasseraustrittsöffnung (2), der äußere Hohlzylinder (4) sowie der innere zentrale Hohlzylinder mit permeabler Wandung (5) im 3D-Druck hergestellt sind.

## Claims

1. Device for the multistage purification of drinking water by combining orthogonal purification techniques in a module, **characterised in that** the device comprises a housing (3), a water inlet opening (1), a water outlet opening (2), an outer hollow cylinder (4) filled with activated carbon and an inner hollow cylinder with a semipermeable wall (5), the inner hollow cylinder (5) comprising a chelating and/or a bactericidal gel for the removal of heavy metals and/or removal of bacteria, the chelating gel and/or the bactericidal gel or both being filled between an outer activated carbon hollow cylinder and a central outlet over the whole length of the hollow cylinder (5), the central outlet consisting of a tube wrapped with a membrane having larger openings than the particle diameter of the surrounding gel with chelating and/or bactericidal effect and wherein the membrane has smaller pores than the particle diameter of the surrounding gel with chelating and/or bactericidal effect.

2. Device according to claim 1, **characterised in that** the central outlet consists of a multiply perforated tube with openings.

3. Device according to claim 2, **characterised in that** the openings of the multiply perforated tube are smaller than the particles of the surrounding gel with chelating and/or bactericidal effect.

4. Device according to claim 1, **characterised in that** the central outlet consists of one or more hollow fibre membranes or hollow fibre membrane bundles.

5. Device according to any one of claims 1 to 4, **characterised in that** the device comprises a pH sensor, conductivity sensor, UV sensor, or sensors for determining the presence of bacteria.

6. Device according to claim 5, **characterised in that** the sensors issue a warning when defined limit values are exceeded or not reached.

7. Device according to one of claims 1 to 6, wherein the device contains further elements, wherein the further elements are selected from a water tank, a softening system, a hot water preparation system, a system for (UV) sterilisation, redox filters, a CO2 addition unit or a chlorination unit.

8. A device according to one of claims 1 to 7, wherein the housing (3), the water inlet opening (1), the water outlet opening (2), the outer hollow cylinder (4) and the inner central hollow cylinder with permeable wall (5) are produced by 3D printing.

## Revendications

1. Dispositif de purification de l'eau potable en plusieurs étapes par combinaison de techniques de purification orthogonales dans un module, **caractérisé en ce que** le dispositive comprend un boîtier (3), une ouverture d'entrée d'eau (1), une ouverture de sortie d'eau (2), un cylindre creux extérieur (4) rempli de charbon actif ainsi qu'un cylindre creux intérieur avec une paroi semi-perméable (5), le cylindre creux intérieur (5) contenant un gel chélatant et/ou bactéricide pour éliminer les métaux lourds et/ou les bactéries, le gel chélatant ou le gel bactéricide ou les deux étant placés entre un cylindre creux extérieur en charbon actif et un écoulement central sur longueur totale du cylindre creux (5), l'écoulement central étant constitué d'un tube enveloppé d'une membrane dont les ouvertures sont plus grandes que le diamètre des particules du gel qui l'entoure et qui a un effet chélatant et/ou bactéricide, et la membrane ayant des pores plus petits que le diamètre des particules du gel qui l'entoure et qui effet chélatant et/ou bactéricide .

2. Dispositif selon la revendication 1, **caractérisé en ce que** le conduit central est constitué d'un tube à plusieurs ouvertures.

3. Dispositif selon la revendication 2, **caractérisé en ce que** les ouvertures du tube à plusieurs ouvertures sont plus petites que les particules du gel à effet chélatant et/ou bactéricide.

4. Dispositif selon la revendication 1, **caractérisé en ce que** le système d'écoulement central est constitué d'une ou de plusieurs membranes à fibres creuses ou membranes à fibres creuses.

5. Dispositif selon l'une des revendications 1 à 4, **caractérisé en ce que** le dispositif comprend un capteur de pH, un capteur de conductivité, un capteur UV ou des capteurs pour la détection de bactéries.

6. Dispositif selon la revendication 5, **caractérisé en ce que** les capteurs émettent un avertissement lorsque les valeurs limites définies sont dépassées ou non atteintes.

7. Dispositif selon l'une des revendications 1 à 6, le dispositif contenant d'autres éléments, les autres éléments sont choisis parmi un réservoir d'eau, une installation d'adoucissement, une installation de production d'eau chaude, une installation de désinfection (UV), des filtres redox, une unité d'ajout de CO2 ou une unité de chloration.

8. Dispositif selon l'une des revendications 1 à 7, dans lequel le boîtier (3), l'orifice d'entrée d'eau (1), l'orifice de sortie d'eau (2), le cylindre creux extérieur (4) ainsi que le cylindre creux central intérieur avec paroi perméable (5) sont fabriqués par impression 3D.
